Europäisches Patentamt

European Patent Office

Office européen des brevets

(19)

(11) Publication number: **0 261 522 B1**

# (12) EUROPEAN PATENT SPECIFICATION

(45) Date of publication of patent specification: **18.12.91**  (51) Int. Cl.⁵: **A61K 35/12**

(21) Application number: **87113308.8**

(22) Date of filing: **11.09.87**

(54) Calcium channel modulating substances.

(30) Priority: **26.09.86 US 912580**
**24.04.87 US 42147**

(43) Date of publication of application:
**30.03.88 Bulletin 88/13**

(45) Publication of the grant of the patent:
**18.12.91 Bulletin 91/51**

(84) Designated Contracting States:
**AT BE CH DE ES FR GB GR IT LI LU NL SE**

(56) References cited:

**TRENDS IN PHARMACOLOGICAL SCIENCES,
vol. 7, no. 5, 1986, pages 171-172, Elsevier
Science Publishers B.V., Amsterdam, NL;
V.RAMKUMAR et al.: "The current status of
the dihydropyridine calcium channel antago-
nist binding sites in the brain"**

**SOCIETY NEUROSCIENCE ABSTRACTS, vol.
11, no. 1, 1985, page 646, no. 192.10;
P.SIEKEVITZ et al.: "Existence of a voltage-
dependent Ca2 + channel protein in synap-
tic membrane (SM) and postsynaptic den-
sity (PGD) fractions isolated from cerebral
cortex (CTX) and cerebellum (CL) of canine
brain, as determined by nitrendipine bind-**
ing"

(73) Proprietor: **MILES INC.**
**1127 Myrtle Street**
**Elkhart Indiana 46514(US)**

(72) Inventor: **Janis, Ronald A.**
**656 High Ridge Road**
**Orange Connecticut 06477(US)**

(74) Representative: **Dänner, Klaus, Dr. et al**
**c/o Bayer AG Konzernverwaltung RP Paten-
tabteilung**
**W-5090 Leverkusen 1 Bayerwerk(DE)**

## Description

Janis et al (J. Clin. Pharmacol., 23, 266-273, 1983) have reported that small quantities of human and rat serum inhibited the binding of the calcium channel antagonist, nitrendipine leading to speculation of the existence of one or more endogenous materials which may modify the activity of calcium channels.

S. A. Thayer et al (9th International Congress of Pharmacology Abstracts, 1984) disclose a soluble crude fraction from brain homogenates which is said to perhaps be associated with calcium channel function. This fraction is described as being able to inhibit the specific binding of [3H]nitrendipine to a high affinity binding site; this binding is said to be non-competitive and is modulated by divalent cations. Said fraction is described as having a molecular weight of between 5,000 and 10,000 daltons, is heat labile and is not destroyed by trypsin. It is further reported by Thayer et al that this crude material produces alterations in the contractile response of the guinea pig ileum to carbachol which suggests that the material may modulate transmembrane calcium fluxes.

Additionally, preliminary reports by Hanbauer and Sanna (Clinical Neuropharmacology, 9, Supp. 4, 220-222 1986) and Ebersole et al (Fed. Proc., 46, 394, 1987) suggest the presence of some 1,4-dihydropyridine inhibiting substance in brain. However, none of the above publications report evidence of any direct effects of said substances on calcium channels nor do those substances have physical characteristics similar to those of the instant claimed invention.

## SUMMARY OF THE INVENTION

The present invention is directed, in one aspect, to an endogenous calcium channel modulating substance characterized in that said substance is obtained from mammalian brain or pituitary gland tissue by extracting said tissue with a solution of about 1 normal HCl, 1% trifluoroacetic acid, 5% formic acid and 1% NaCl. On gel filtration with a Waters Protein-Pak® 125 column said substance has the characteristics of peptides having a molecular weight of approximately 18,000 to 25,000 daltons; elutes from a $C_{18}$ reverse phase column at approximately 31-33% acetonitrile using gradient elution in 0.1% trifluoroacetic acid. The activity of said substance elutes from hydroxylapatite (Bio Gel HT® from Bio-Rad) between 0.1 and 0.2 molar sodium phosphate (pH 7) and has an approximate molecular weight on sodium dodecylsulfate gels of 18,000 to 20,000. The substance inhibits the binding of the tritiated calcium channel antagonist PN 200-110 to cardiac and brain membranes; the activity is stable to boiling; is sensitive to a protease from Streptomyces griseus (Cat. No. 165-921, Boehringer-Mannheim Biochemicals, Indianapolis, Indiana USA); inhibits the slowly inactivating $Ca^{2+}$ channel in rat $GH_3$ anterior pituitary cells in a time and voltage-dependent manner when added to the inside of the cell; and the exhibited inhibitory activity on dihydropyridine receptor binding is reversed by 10 millimolar $Ca^{2+}$ or by dilution in the presence of 200 millimolar KCl.

In a second aspect, the present invention is directed to an endogenous calcium channel modulating substance characterized in that said substance is obtained from mammalian stomach tissue by extracting said tissue with a solution of about 9% HCl, 1% trifluoroacetic acid, 5% formic acid and 1% NaCl. On gel filtration with a Bio-Rad Bio-Sil TSK® -250 column in 40% acetonitrile, said substance has the characteristics of a peptide having a molecular weight of approximately 7,000 to 10,000 daltons; elutes from a Waters μBondapak $C_{18}$ reverse phase analytical Radial-pak column at approximately 47% acetonitrile using gradient elution in 0.1% trifluoroacetic acid. The substance inhibits the binding of the tritiated calcium channel antagonist PN 200-110 to cardiac membranes and reversibly inhibits the slowly-inactivating $Ca^{2+}$ channel in rat $GH_3$ anterior pituitary cells in a time- and voltage-dependent manner when added to the inside of the cell. Its effect on the calcium channel is reversed by hyperpolarization of the cell membrane.

## BRIEF DESCRIPTION OF THE DRAWINGS

Figure 1 represents the location of active fractions (hatched area) isolated from calf brain (Example 1).

Figure 2 represents the effect of various amounts of the endogenous calcium channel modulating substance of Example 1 on the binding of [3H]1,4-dihydropyridine to membranes from skeletal and cardiac muscle and brain.

Figure 3 graphically represents the rate of onset of calcium channel block by the endogenous calcium modulating substance of Example 1.

Figure 4 graphically represents the rate of onset of calcium channel block by the endogenous calcium channel modulating substance of Example 3.

## DETAILED DESCRIPTION OF THE INVENTION

The endogenous substances of the present invention may be isolated from mammalian tissues containing said substances by techniques which have general applicability to the extraction and isolation of endogenous substances from mammalian tissues or fluids. In particular, the calcium channel modulating substances of the present invention are obtained from mammalian tissue or fluid by acid extraction followed by molecular weight exclusion filtration to obtain the desired material. This material is then chromatographed on a preparative reverse-phase column using an acetonitrile/trifluoroacetic acid solution as the eluent. The various fractions are then collected and dried and assessed for activity as, for example, by measuring the inhibition of [³H]PN 200-110 (see *infra*) to rat heart membranes by the method of Janis et al, *supra*. The endogenous calcium channel modulating substances of the present invention are preferably, though not necessarily exclusively, isolated from mammalian brain and stomach tissue. Said endogenous substance is isolated from the tissue as substantially purified material which did not heretofore exist in such form in the tissue from which it was obtained.

As noted previously, these endogenous substances act on calcium channels to modulate the activity thereof. Accordingly, said substances find utility in applications where the calcium channel antagonists in general are currently being used. These applications include various cardiovascular indications such as angina pectoris (vasospastic and chronic stable), atrial flutter and fibrillation, cardioplegia, hypertension, myocardial ischemia and failure, peripheral vascular disease; cerebral insufficiency; migraine; subarachnoid hemorrhage; asthma; esophageal motor disorders, among others. Of course, the endogenous substances may also find therapeutic efficacy in other disease states.

The following examples are provided as a means of illustrating the present invention.

### EXAMPLE 1

Isolation and Analysis of Endogenous Substance From Calf Brain

#### a) Extraction

Fifty calf brains from a local slaughterhouse (or, alternatively, 1 kilogram of lyophilized calf brain available from Burlington Bio-Medical Corp., New York) were homogenized with a PT45® probe (Brinkman Instruments, New York) for 2 minutes at setting 6 in 21 liters (l) of the following extraction solution (Quirion et al, Peptides 5:967-973, 1984): 9% HCl, 5% formic acid, 1% trifluoroacetic (TFA) acid, and 1% NaCl. The homogenate was centrifuged for 45 minutes at 16,000 x g resulting in 19 l of material which was extracted 2 times with petroleum ether at a ratio of 2:1 (ether:sample). The aqueous phase from this extraction (13 l) was adjusted to pH 2 with NaOH and subjected to ultrafiltration with a Pellicon® (Millipore) apparatus using a 30,000 molecular weight exclusion filter. The fraction of less than 30,000 daltons was chromatographed as follows.

#### b) Chromatography

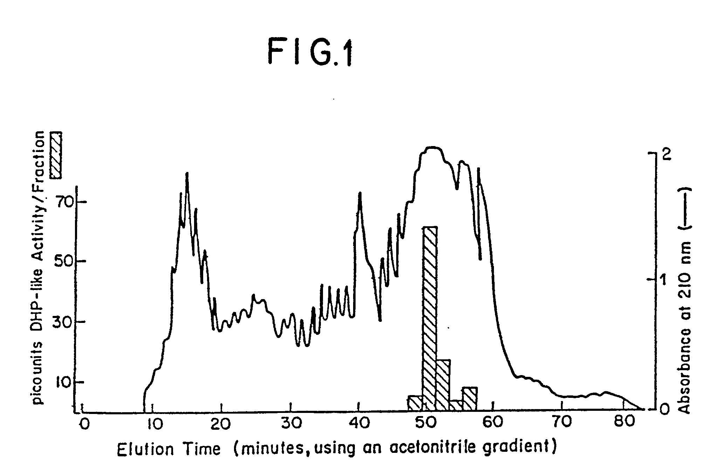

The fraction of less than 30,000 daltons was subjected to reverse-phase chromatography on a Waters C₁₈ reverse phase preparative column (5.7 x 30 centimeters) and run in a Waters Prep 500A® HPLC system. The fraction was loaded onto the column (which had been previously equilibrated in 0.1% TFA) through the pump at a rate of 100 milliliters per minute (ml/min). The column was washed with 0.1% TFA to remove salts and other non-binding material and then eluted with a 90 minute acetonitrile gradient (0-70%) in 0.1% TFA at 0.62% acetonitrile per minute at 150 ml/min. Forty-six 300 milliliter (ml) fractions were collected and 10 ml aliquots of each were dried in a Speed Vac® (Savant Instruments, New York). The activity eluted from the C₁₈ column (as subsequently determined in the ligand binding assay and confirmed by electrophysiology, *infra*) at approximately 31-33% acetonitrile and is graphically represented as the hatched area in Figure 1.

Hydroxylapatite (Bio Gel HT® commercially available from Bio-Rad, Inc.) was washed and equilibrated to pH 7 in 0.01 molar (M) sodium phosphate. 3-5 milligrams (mg) of the active brain fractions obtained from the reverse phase chromatography, above, were dissolved in the above sodium phosphate buffer and adjusted to pH 7 with NaOH. Any precipitate which resulted was removed by centrifugation and the resultant supernatant (1 ml representing 3-5 mg protein) was applied to the hydroxylapatite column. Fractionation on said column showed a peak of activity (as subsequently determined) which eluted between 0.1 and 0.2 M sodium phosphate (pH 7). The activity appeared to be associated with the approximately 18,000 to 20,000 dalton proteins as determined on sodium dodecylsulfate gels.

This 18,000 to 20,000 dalton material eluted from a Waters SP-5PW® cation-exchange column between 0.4 and 0.55 M NaCl using a NaCl gradient from 0.01 M to 0.8 M in 75 minutes which contained 10% acetonitrile and 20 millimolar (mM) Tris at pH 7.5. Chromatography of said material (obtained from the reverse phase chromatography) on a Waters DEAE-5PW® anion-exchange column previously equilibrated in 0.02 M NaCl, 0.02 M Tris pH 8, showed that the activity did not bind to the column when the sample was injected in a solution of the same composition. Chromatography of the active brain fractions (obtained from the reverse phase chromatography) on a Waters Protein-Pak® 125 gel filtration column showed that the activity eluted in a volume range equivalent to 18,000 to 25,000 daltons when using a mobile phase containing 40% acetonitrile, 0.1% TFA. The purified mixture of 18,000 and 20,000 dalton proteins from the SP-5PW® cation-exchange column was further purified to apparent homogeneity on a Waters μBondapak® $C_{18}$ (8 mm x 10 cm) Radial-Pak cartridge. Said proteins eluted at 32% and 33% acetonitrile respectively, when eluted with the following gradient: 0-26.4% acetonitrile in 20 minutes and then 26.4 to 40% acetonitrile in 27 minutes. The gradient contained 0.1% TFA throughout.

### c) Ligand binding assay

Preparation of microsomes and ligand binding was carried out essentially as described previously by Janis et al, *supra*. Frozen rat hearts (Pel-Freeze) were thawed in 50 mM HEPES buffer (i.e., N-2-hydroxyethylpiperazine-N'-2-ethanesulfonic acid, free acid), pH 7.0, and the atria removed. 5 grams (g) of ventricular myocardium were minced with scissors and homogenized in 50 ml HEPES buffer with a Brinkman Polytron PT20® probe at setting 7 for 10 seconds. This was repeated 2 times with a 10 second cooling on ice between each homogenization. The homogenate was centrifuged at 5,000 x g for 10 minutes at 4° Centigrade (C). The supernatant fraction was removed and centrifuged at 48,000 x g for 30 minutes. The resultant microsomal pellet was resuspended in buffer and subsequently used in the assay as described below. Protein was determined by the method of Bradford (Anal. Biochem. 72:248-253, 1976) using bovine serum albumin as standard.

The activity of the fractions isolated by chromatography was defined against a standard curve for competition between the dihydropyridine calcium channel antagonist nimodipine and the calcium channel antagonist, isopropyl 4-(2,1,3-benzoxadiazol-4-yl)--1,4-dihydro-2,6-dimethyl-pyridine-3,5-dicarboxylic acid 1-methyl ester (known as PN 200-110 and referred to hereafter as [³H]DHP). Said standard curve was derived from data gained by incubating nimodipine and $2 \times 10^{-10}$ M [³H]DHP and microsomal protein per 0.25 ml of assay in 50 mM HEPES buffer (pH 7.0) at 25°C for 45 minutes and 37°C for 30 minutes. A cocktail of protease inhibitors ($1 \times 10^{-6}$ M aprotinin, $5 \times 10^{-5}$ M benzamidine and $5 \times 10^{-5}$ M leupeptin) was used but did not inhibit control binding. Said cocktail of protease inhibitors is used to prevent breakdown of the isolated fractions during the assay by proteases which may be present in the membranes or fractions themselves and to exclude the possibility that proteases sensitive to these inhibitors contributed to the activity of the fraction. Following incubation, the bound drug was separated from the free by rapid filtration through Whatman GF/B® filters using a Brandel Harvester, followed by two consecutive 1 ml buffer washes at 22°C. The filters were placed in scintillation vials with 5 ml of liquid scintillation fluid. Samples were counted in a Beckman LS 1800® liquid scintillation counter at an efficiency of approximately 55%. Nonspecific binding was defined as the amount of radioligand (i.e., [³H]DHP) bound in the presence of nimodipine, which was 10% of total binding.

The fractions isolated from brain were evaluated in the ligand binding assay substantially as described above. Fractions were dissolved in 50 mM HEPES (pH 7.0) and protein concentration was determined by either visible or UV absorption. The pH was carefully monitored and maintained at 7.0 by the addition of 1M HEPES. All fractions were centrifuged and the supernatants assayed. Binding experiments were performed under sodium vapor light.

### d) Results

Five fractions eluted from the reverse phase column in the approximately 30-34% acetonitrile range (as described in Example 1b, above and depicted in Figure 1) showed activity in the ligand binding assay. The combined activity represented 95 picounits (pU) of total activity, where one pU of activity is defined as being equivalent to the amount of inhibition caused by one picomole of nimodipine which gave equivalent results. The active fractions contained up to 2.5 pU/mg protein from the first chromatographic step. All activity was reversed with the addition of 10 mM $Ca^{2+}$ or by dilution in the presence of 200 mM KCl, meaning that the active fractions acted reversibly and therefore did not represent an enzymatic reaction.

4

In order to further determine the specificity of action of a particular fraction (i.e., to insure that the fraction was acting competitively on the dihydropyridine receptor and not simply depleting the free ligand), a determination of the amount of free ligand was done by dialysis as follows.

A 12 millimeter (mm) segment of Spectra-por® 7 dialysis tubing (0.46 ml/cm) was washed thoroughly with water to remove EDTA and other chemicals. The molecular weight cut-off of this tubing was 1,000. Dialysis was performed in 16 mm glass tubes placed in a shaker incubator at 25°C overnight. The volume inside the bag (made from closing the tubing by tying it) was 2.5 ml; the outside volume was 10 ml of 50 mM HEPES buffer, pH 7.0. Experiments were done with the [3H]DHP present both inside and outside the bag. The concentration of the [3H]DHP ligand used was always 0.2 nM. A control using 5 mg bovine serum albumin (BSA) inside the bag was carried out. BSA is known to bind [3H]DHP ligand hydrophobically (which is evident because of the increased counts per minute (cpm) associated with BSA inside rather than outside the bag). The brain fractions tested were also 5 mg by weight. Dialysis was carried out in the presence of the cocktail of protease inhibitors described *supra* present both inside and outside the bag. The experiment was terminated by taking representative aliquots from inside and outside the bag (in triplicate). The cpm were then adjusted for total volume. No [3H]DHP ligand was bound to the fractions tested, demonstrating that the inhibitory activity in the ligand binding assay was not due to a decrease in the free [3H]DHP concentration.

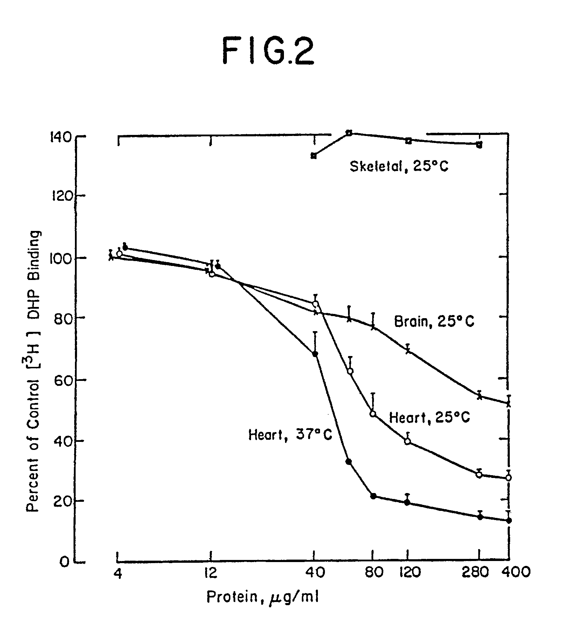

In further analyses, this endogenous calcium channel modulating substance maintained its inhibitory activity on dihydropyridine receptors when boiled for 30 minutes. This activity was reversed by the presence of 10 mM $Ca^{2+}$, demonstrating reversibility of action. Further, after said substance was heated at 270°C for 3 hours (in order to ash it), activity was lost further indicating that the substance is organic in nature rather than inorganic (a control of 0.8 M NaCl maintained activity upon ashing). The effect of the endogenous calcium channel modulating substance varied on brain, heart and skeletal muscle membranes (Figure 2). The substance caused more inhibition in heart than brain membranes and showed stimulation in skeletal muscle membranes. This observed differential activity was further evidence of a specific interaction with the dihydropyridine binding site rather than the non-specific reduction of free ligand concentration.

e) Electrophysiology

In order to further demonstrate that the endogenous calcium channel modulating substance of the present invention inhibits the $Ca^{2+}$ channel, the following study was undertaken (as described by Cohen and McCarthy, J. Physiol., in press).

A 150 mg sample of the calcium channel modulating substance of the present invention was dissolved in 37 ml of 0.05% TFA and 37 ml of methanol. This mixture was shaken together with 150 ml of chloroform in a glass separatory funnel for 10 minutes and then allowed to stand for 1 hour to achieve separation. The upper aqueous phase was collected and freeze-dried. The dried fraction was dissolved in 160 ml of 50 mM HEPES; ultrafiltrated through a 5,000 dalton filter in an Amicon cell and then washed with 2 more washes of 160 ml each of 150 mM HEPES. The retentate yielded 10 pU/mg protein activity and was used for electrophysiology as described below. The fraction was passed through a 5,000 dalton filter in an Amicon cell to remove low molecular weight substances. The retentate retained 90% of the inhibitory activity showing dose-dependent inhibition up to 100 μg protein. Since the majority of the activity was over 5,000 daltons, this step removed any interfering substances and salts under approximately 5,000 daltons.

Rat anterior pituitary (GH3) cells were bathed in Tyrode's solution containing NaCl (150 mM), KCl (4.0 mM), $CaCl_2$ (9.0 mM), $MgCl_2$ (0.5 mM), dextrose (5 mM) and HEPES (10 mM, pH 7.5). The tip of a patch electrode was filled with a solution containing CsCl (108 mM), tetrabutylammonium chloride (10 mM), BAPTA, i.e., 1,2-bis-(2-aminophenoxy)ethane-N,N,N',N'-tetra-acetic acid (11 mM), $CaCl_2$ (0.9 mM), $MgCl_2$ (6 mM), adenosine triphosphate (5 mM) and HEPES (20 mM, pH 7.2). This solution is designed to eliminate all potassium channel currents and to slow rundown of the calcium channel currents. After the initial filling of the tip, the patch electrode was back-filled with the above-described solution plus the dissolved endogenous calcium channel modulating substance at a concentration of 0.5 mg/ml. The patch electrode was then mounted under positive pressure to allow both mixing and diffusion of drug into the tip. A high resistance seal was made to the cell by applying negative pressure and subsequently, the membrane beneath the electrode was disrupted by an increase in negative pressure. Recordings of inward calcium channel currents were performed in a bath solution of tetraethylammonium chloride (117 mM), $BaCl_2$ (20 mM), $MgCl_2$ (0.5 mM), tetrodotoxin ($2 \times 10^{-4}$ mM), dextrose (5 mM), sucrose (32 mM) and HEPES (10 mM, pH 7.5).

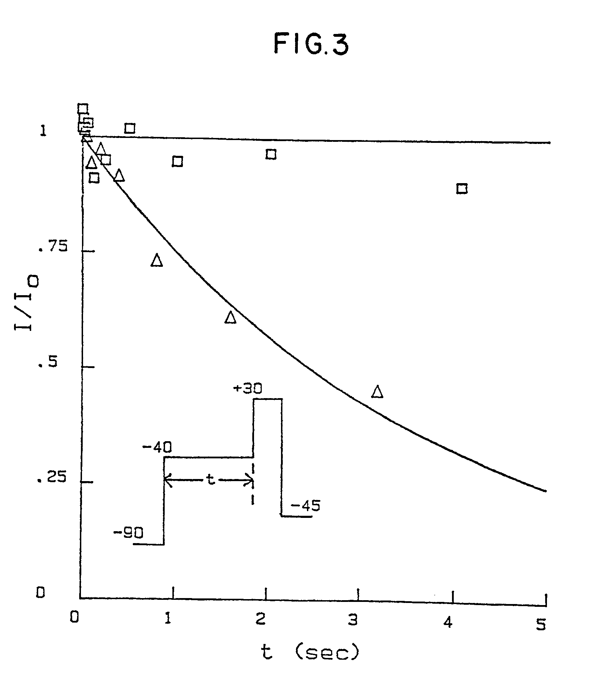

Time and voltage-dependent block of the slowly inactivating calcium channels by intracellular addition of the endogenous substance of the present invention was obtained in four experiments using three separate fractions. It is known that GH3 rat anterior pituitary cells exhibit little or no inactivation for potentials more negative than -20 millivolts (mV). Over this range, where in control studies there is no inactivation, the endogenous calcium channel modulating substance produced potent time and voltage-dependent block of the dihydropyridine-sensitive calcium channels. The onset of block at variable prepulse durations to more depolarized potentials produced potent calcium channel block. It was also shown that said substance has no effect on the voltage-dependence of activation or inactivation of the transient calcium channels which are believed to be available for opening in the steady state. These results are graphically represented in Figure 3.

Figure 3 shows the rate of onset of calcium channel block by the endogenous substance of the present invention. The time course of block of the slowly-inactivating channels was determined by tail current analysis as described in Matteson and Armstrong (J. Gen. Physiol. 87:161-182, 1986) and Cohen and McCarthy (J. Physiol., in press). A protocol was used which takes advantage of the fact that GH₃ cells show little inactivation of inward calcium channel current during prolonged test pulses. For each data point, the membrane potential was held at -90 mV and then stepped to the prepulse potential ($V_p$ = -40 mV) for a variable duration prepulse (t). Then a 10 millisecond (ms) test pulse to +30 mV produced maximal activation of the slowly-inactivating Ca channels and tail currents were measured upon repolarization ($V_r$ = -45 mV) as shown in the inset of Figure 3. During the control prepulses (open squares), the fast component of tail current corresponding to the slowly-inactivating channels showed little or no inactivation. The data are fit as a normalized mean of 12 points (104.5 picoamps, pA). With increasing prepulse duration, the endogenous calcium channel modulating substance was added to the pipette (open triangles) and produced time-and voltage-dependent block of the calcium channel. The 8 data points of said substance are fit to the equation:

$$I = (I_o - I_\infty) \exp(-t/\tau \text{onset}) + I_\infty. \text{ For } Vp = -40 \text{ mV,}$$
$$I_o = 143.17 \text{ pA, } \tau \text{onset} = 3.56 \text{ sec.}$$

The values were normalized by taking the ratio of the fast tail current amplitudes fit by a single exponential (I) to the value obtained by a curve fitting these data points using a non-linear test squares analysis (Colquhoun, Lectures on Biostatistics, Oxford University Press, London) and extrapolating back to time zero ($I_o$). These normalized values ($I/I_o$) were plotted as the ordinate. The cycle length was ten seconds. The potent time and voltage-dependent, reversible block of the calcium channels demonstrate that the 18,000 to 20,000 dalton endogenous substance blocks calcium channels and inhibits dihydropyridine binding. Since said substance inhibits dihydropyridine binding, it is likely that both also inhibit calcium channels. The lack of effect on another type of calcium channel in these cells shows specificity of action for slowly-inactivating calcium channels.

While this endogenous calcium channel modulating substance is described as being a single material having a molecular weight on sodium dodecylsulfate gels of 18,000 to 20,000, the skilled artisan will appreciate that upon further characterization, what appeared to be one material may in fact be two or more. Thus, the material described herein as having a molecular weight of 18,000 to 20,000 may actually be two substances of 18,000 and 20,000 molecular weight, respectively. Such differences may be accounted for by the complexation of heavy metal ions, changes in structural conformation, and the like.

Further, a low molecular weight substance that inhibits DHP binding was isolated from the active fraction (31-33% acetonitrile) obtained from the preparative column procedure described in Example 1b, *supra*. When this fraction was chromatographed on either a Waters Protein Pak-125® or a TSK-250® gel filtration column using 40% acetonitrile in 0.1% TFA as the mobile phase, the activity (as determined in ligand binding assays described in Example 1c) eluted in a volume less than β-lipoprotein fragment (amino acids 61-63) having a molecular weight of 443. This activity of this described low molecular weight substance (less than 600 daltons) from brain was not reversed by 10 mM $Ca^{2+}$ or 4 μM EDTA; it was stable to boiling; and it cross-reacted with antibodies to nitrendipine (said antibodies prepared, and cross-reactivity determined as described by Campbell et al, Proc. Natl. Acad. Sci. USA 83:2792-2796, 1986). The latter result, coupled with the inhibition of dihydropyridine binding strongly suggests that this low molecular weight substance is an endogenous dihypropyridine-like substance.

EXAMPLE 2

Isolation of Endogenous Substance From Sheep Pituitary

a) Extraction

Lyophilized sheep pituitary (100 g) was obtained from Burlington Bio-Medical Corp., New York. The sheep pituitary was homogenized in 1 l of acid extraction solution (i.e., 1 M HCl, 5% formic acid, 1% TFA and 0.5% NaCl) using a polytron PT20® probe in batches of 500 ml each. The homogenate was centrifuged at 16,000 x g for 75 minutes. The supernatant was filtered through 4 layers of cheesecloth and the filtrate extracted 2 times with petroleum ether (2:1, ether:sample) to remove lipids. The aqueous phase from the extractions were combined (about 600 ml) and then diluted to 1 l with the acid extraction solution and then filtered through a 30,000 dalton cut-off limit Minitan® ultrafiltration apparatus (Millipore). The fraction of less than 30,000 daltons (860 ml having a protein concentration of 1 mg/ml) was then chromatographed as described below.

b) Chromatography

The fraction of Example 2a was subjected to reverse-phase chromatography utilizing the same methodology as described in Example 1b. The column was eluted with a 70 minute linear gradient of from 0-80% acetonitrile in 0.1% TFA. Forty-six 200 ml fractions were collected and 10 ml aliquots of each were dried in a Speed Vac® (Savant Instruments, New York). The fractions numbered 34-38 which showed the highest activity were pooled (representing a total volume of 340 ml) and lyophilized or were diluted out with weak buffer and reloaded onto a subsequent reverse phase column for repurification. The fractions were assayed for inhibition of dihydropyridine binding to rat heart membranes as follows.

c) Ligand binding assay

The ligand binding assay described in Example 1c was repeated with the endogenous fractions isolated by chromatography in Example 2b.

d) Results

Activity was observed in the 30-40% acetonitrile range. This activity was maintained in the presence of 4 $\mu$M EDTA. Approximately 50% of the inhibitory activity of the fractions was lost in the presence of 100 $\mu$M $Ca^{2+}$. A time course of 45 and 90 minutes did not increase the percent inhibition of any fractions. A 5,000 dalton retentate (prepared as described in Example 1) showed a dose-dependent inhibition up to 100 $\mu$g protein. The endogenous substance showed tissue selectivity with brain, heart and skeletal muscle membranes.

Cation exchange chromatography on a Waters SP-5PW® column produced a peak of inhibitory activity at 0.4 M ammonium acetate when eluted with a 0.01 to 1.0 M 60 minute gradient at pH 5.5. When chromatographed on a Waters DEAE-5PW® anion exchange column using a 60 minute gradient of 0.01 to 0.5 M NaCl (pH 8), the inhibitory activity eluted near the void volume and stimulatory activity eluted at 0.3 M NaCl. These results suggest that at least some of the activity shown by the endogenous substance isolated from the pituitary is due to the same substance as that isolated from brain.

EXAMPLE 3

Isolation and Analysis of Endogenous Substance From Lamb Abomasum

a) Extraction

Three hundred grams of lyophilized lamb abomasum (available from Burlington Bio-Medical Corp., New York) were homogenized with a PT45® probe (Brinkman Instruments, New York) for 2 minutes at setting 6 in 6 liters (l) of the following extraction solution (Quirion et al, Peptides 5:967-973, 1984): 9% HCl, 5% formic acid, 1% trifluoroacetic (TFA) acid, and 1% NaCl. The homogenate was centrifuged for 45 minutes at 16,000 x g resulting in 5 l of material which was extracted 1 time with petroleum ether at a ratio of 2:1 (ether:sample). The aqueous phase from this extraction (4.8 l) was adjusted to pH 2 with NaOH and subjected to ultrafiltration with a Pellicon® (Millipore) apparatus using a 30,000 molecular weight exclusion filter. The fraction of less than 30,000 daltons was chromatographed as follows.

b) Chromatography

The fraction of less than 30,000 daltons was subjected to reverse-phase chromatography on a Waters

$C_{18}$ reverse phase preparative column (2.5 x 30 centimeters) and run in a Waters Prep 500A® HPLC system. The fraction was loaded onto the column (which had been previously equilibrated in 0.1% TFA) through the pump at a rate of 100 milliliters per minute (ml/min). The column was washed with 0.1% TFA to remove salts and other non-binding material and then eluted with a 90 minute acetonitrile gradient (0-70%) in 0.1% TFA at at 100 ml/min. Fifty 200 milliliter (ml) fractions were collected and 5 ml aliquots of each were dried in a Speed Vac® (Savant Instruments, New York). The activity eluted from the $C_{18}$ column (as subsequently determined in the ligand binding assay and confirmed by electrophysiology, *infra*) at approximately 43-48% acetonitrile.

The active fractions obtained from the preparative reverse phase chromatography were further purified on a Waters μBondapak $C_{18}$ analytical reverse phase Radial-pak® (8 mm x 10 cm). The active component eluted from the column at 47% acetonitrile in the following gradient in 0.1% TFA: 0-40% in 32 minutes, 40-70% in 40 minutes.

The highly purified activity from the analytical reverse phase column was chromatographed on a Bio-Rad Bio-Sil TSK-250® column using 40% acetonitrile, 0.1% TFA as the mobile phase. A single peak of absorbance at 214 nanometers (nm) elutes from the column in a volume range equivalent to 7,000 to 10,000 daltons. This position is between the two peptide standards, cytochrome C and aprotinin, which are 12,500 daltons and 6,500 daltons, respectively. The amino acid composition based on a molecular weight of 8,000 was estimated as follows:

| Alanine | 4 | Leucine | 6 |
|---|---|---|---|
| Arginine | 5.5 | Lysine | 7.8 |
| Asparagine or | | Methionine | 2.3 |
| aspartic acid | 5.6 | Phenylalanine | 2.6 |
| Cysteine | N/A | Proline | 4 |
| Glutamine or | | Serine | 5.6 |
| glutamic acid | 8.2 | Threonine | 2.9 |
| Glycine | 5 | Tryptophan | N/A |
| Histidine | N/A | Tyrosine | 2 |
| Isoleucine | 5 | Valine | 4 |

The Partial amino acid sequence from the N-terminus was estimated as follows:

```
(1)                                            (10)
(Gly)-Leu-Leu-?-Gly-Pro-(Pro)-(Arg)-Lys-Ile-Ile-Lys-
                           (19)              (24)
Ser-Leu-Glu-Asp-Met-Val-Gly-(Asp)-Gln-Pro-Asn-Glu.
```

c) Ligand binding assay Preparation of microsomes and ligand binding was carried out essentially as described previously by Janis et al, *supra*. Frozen rat hearts (Pel-Freeze) were thawed in 50 mN HEPES buffer (i.e., N-2-hydroxy-ethylpiperazine-N'-2-ethanesulfonic acid, free acid), pH 7.0, and the atria removed. 5 grams (g) of ventricular myocardium were minced with scissors and homogenized in 50 ml HEPES buffer with a Brinkman Polytron PT20® probe at setting 7 for 10 seconds. This was repeated 2 times with a 10 second cooling on ice between each homogenization. The homogenate was centrifuged at 5,000 x g for 10 minutes at 40 Centigrade (C). The supernatant fraction was removed and centrifuged at 48,000 x g for 30 minutes. The resultant microsomal pellet was resuspended in buffer and subsequently used in the assay as described below. Protein was determined by the method of Bradford (Anal. Biochem. 72:248-253, 1976) using bovine serum albumin as standard.

The activity of the fractions isolated by chromatography was defined against a standard curve for competition between the dihydropyridine calcium channel antagonist nimodipine and the calcium channel antagonist, isopropyl 4-(2,1,3-benzoxadiasol-4-yl)-1,4-dihydro-2,6-dimethyl-pyridine-3,5-dicarboxylic acid 1-methyl ester (known as PN 200-110 and referred to hereinafter as [3H]DHP). Said standard curve was derived from data gained by incubating nimodipine and $2 \times 10^{-10}$ M [3H]DHP and microsomal protein per 0.25 ml of assay in 50 mM HEPES buffer (pH 7.0) at 25° C for 45 minutes and 37° C for 30 minutes. A cocktail of protease inhibitors ($1 \times 10^{-6}$ M aprotinin, $5 \times 10^{-5}$ M benzamidine and $5 \times 10^{-5}$ M leupeptin) was used but did not inhibit control binding. Said cocktail of protease inhibitors was used to prevent breakdown of the isolated fractions during the assay by proteases which may be present in the membranes or fractions themselves and to exclude the possibility that proteases sensitive to these inhibitors contributed to the activity of the fraction. Following incubation, the bound drug was separated from the free by rapid filtration through Whatman GF/B® filters using a Brandel Harvester, followed by two consecutive 1 ml buffer washes at 22° C. The filters were placed in scintillation vials with 5 ml of liquid scintillation fluid. Samples were counted in a Beckman LS 1800® liquid scintillation counter at an efficiency of approximately 55%. Nonspecific binding was defined as the amount of radioligand (i.e., [3H]DHP bound in the presence of nimodipine, which was 10% of total binding. The fractions isolated from stomach were evaluated in the ligand binding assay substantially as described above. Fractions were dissolved in 50 mM HEPES (pH 7.0) and protein concentration was estimated by UV absorption. The pH was carefully monitored and maintained at 7.0 by the addition of 1 M HEPES. All fractions were centrifuged and the supernatants assayed. Binding experiments were performed under sodium vapor light.

d) Results

Ligand Binding

Fractions eluting from the reverse phase column in the approximately 43-48% acetonitrile range showed activity in the ligand binding assay (16 to 29% inhibition of DHP binding) Re-chromatography of these on the analytical reverse phase column resulted in the active fraction eluting at 47% acetonitrile.

Approximately 23 $\mu$g of this protein produced 51% inhibition of [3H]DHP binding. Chromatography of this on Bio-Rad Bio-Sil TSK 250® resulted in the active fraction eluting with an apparent size of 7,000 to 10,000 daltons. Approximately 100 $\mu$g of this fraction gave 56% inhibition in the DHP binding assay.

e) Electrophysiology

In order to further demonstrate that this endogenous calcium channel modulating substance inhibits the $Ca^{2+}$ channel, the following study was undertaken (as described by Cohen and McCarthy, J. Physiol., in press).

Rat anterior pituitary (GH3) cells were bathed in Tyrode's solution containing NaCl (150 mM), KCl (4.0 mM), $CaCl_2$ (9.0 mM), $MgCl_2$ (0.5 mM), dextrose (5 mM) and HEPES (10 mM, pH 7.5). The tip of a patch electrode was filled with a solution containing CsCl (108 mM), tetrabutylammonium chloride (10 mM), BAPTA, i.e., 1,2-bis-(2-aminophenoxy)ethane-N,N,N',N'-tetraacetic acid (11 mM), $CaCl_2$ (0.9 mM), $MgCl_2$ (6 mM), adenosine triphosphate (5 mM) and HEPES (20 mM, pH 7.2). This solution is designed to eliminate all potassium channel currents and to slow rundown of the calcium channel currents. After the initial filling of the tip, the patch electrode was back-filled with the above-described solution plus the dissolved endogenous calcium channel modulating substance at a concentration of 1.6 to 5 $\mu$g/ml. The patch electrode was then mounted under positive pressure to allow both mixing and diffusion of drug into the tip. A high resistance seal was made to the cell by applying negative pressure and subsequently, the membrane beneath the electrode was disrupted by an increase in negative pressure. Recordings of inward calcium channel currents were performed in a bath solution of tetraethylammonium chloride (117 mM), $BaCl_2$ (20 mM), $MgCl_2$ (0.5 mM), tetrodotoxin ($2 \times 10^{-4}$ mM), dextrose (5 mM), sucrose (32 mM) and HEPES (10 mM, pH 7.5).

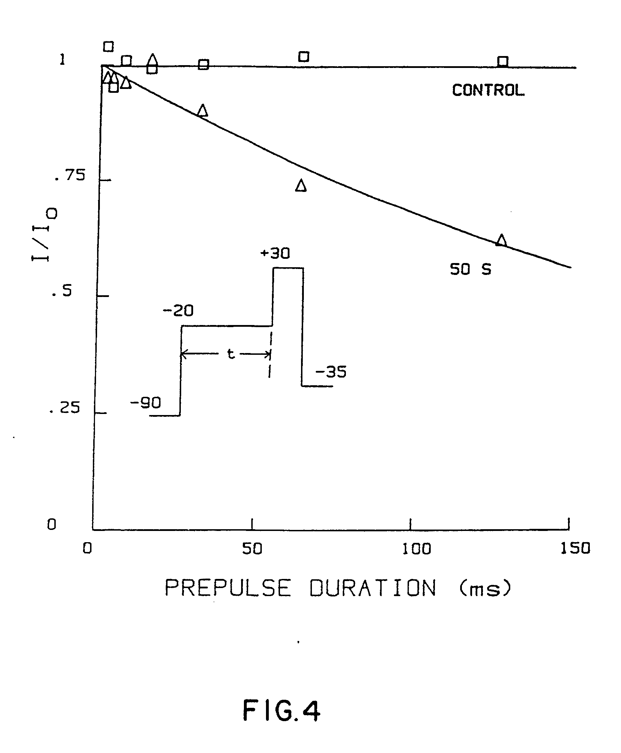

Time and voltage-dependent block of the slowly inactivating calcium channels by intracellular addition of the endogenous substance of the present invention was obtained in seven experiments using two separate fractions. It is known that GH3 rat anterior pituitary cells exhibit little or no inactivation for potentials more negative than -20 millivolts (mV). Over this range, where in control studies there is no inactivation, the endogenous calcium channel modulating substance produced potent time and voltage-dependent block of the dihydropyridine sensitive calcium channels. The onset of block at variable prepulse durations to more depolarized potentials produced potent calcium channel block. It was also shown that said substance has no effect on the voltage-dependence of activation or inactivation of the transient calcium channels which are

9

believed to be available for opening in the steady state. These results are graphically represented in Figure 4.

Figure 4 shows the rate of onset of calcium channel block by the endogenous substance of the present invention. The time course of block of the slowly-inactivating channels was determined by tail current analysis as described in Matteson and Armstrong (J. Gen. Physiol. 87:161-182, 1986) and Cohen and McCarthy (J. Physiol., in press). A protocol was used which takes advantage of the fact the $GH_3$ cells show little inactivation of inward calcium channel current during prolonged test pulses. For each data point, the membrane potential was held at -90 mV and then stepped to the prepulse potential ($V_p$ = -20 mV) for a variable duration prepulse (t). Then a 10 millisecond (ms) test pulse to +30 mV produced maximal activation of the slowly-inactivating Ca channels and tail currents were measured upon repolarization ($V_r$ = -35 mV) as shown in the inset of Figure 4. During the control prepulses (open squares), the fast component of tail current corresponding to the slowly-inactivating channels showed little or no inactivation. The data are fit as a normalized mean of 7 points (342.5 picoamps, pA). With increasing prepulse duration, the endogenous calcium channel modulating substance was added to the pipette (open triangles) and produced time-and voltage-dependent block of the calcium channel. The 7 data points of said substance are fit to the equation:

$$I = (I_0 - I_\infty) \exp(-5/\tau \text{onset}) + I_\infty. \text{ For Vp} + -20 \text{ mV,}$$
$$I_0 = 127.05 \text{ pA, } \tau \text{onset} + 0.259 \text{ sec.}$$

The values were normalized by taking the ratio of the fast tail current amplitudes fit by a single exponential (I) to the value obtained by a curve fitting these date points using a non-linear test squares analysis (Colguhoun, Lectures on Biostatistics, Oxford University Press, London) and extrapolating back to time zero ($I_0$). These normalized values ($I/I_0$) were plotted as the ordinate. The cycle length was ten seconds.

EXAMPLE 4

Utilizing the techniques described in the above Examples 1, 2 and 3, endogenous calcium channel modulating substances have also been isolated from calf stomach (abomasum) and blood. The material from calf abomasum (less than 30,000 dalton fraction) was subjected to reverse phase chromatography on a Waters $C_{18}$ preparative column and eluted at approximately 30% acetonitrile using gradient elution in 0.1% TFA. Size exclusion chromatography resulted in two fractions, the larger of which exhibited characteristics similar to those of the endogenous substance isolated in Example 1. Another active fraction from calf abomasum eluted at 40-46% acetonitrile and was less than 1,000 daltons in size.

Similarly, the material isolated from blood (a less than 30,000 dalton fraction) was chromatographed on a Waters semi-preparative $C_{18}$ column, and said material eluted at approximately 38% acetonitrile using gradient elution in 0.1% TFA. When this material was chromatographed on a TSK-250® gel filtration column using 40% acetonitrile in 0.1% TFA as the mobile phase, the activity (as determined in ligand binding assays) eluted in the same volume as a $\beta$-lipotropin fragment (amino acids 61-64) which has a molecular weight of 443. The inclusion volume measured by injecting NaCl was more than 10% of an elution range away from this position, indicating this size is reasonably accurate.

**Claims**

**Claims for the following Contracting States : AT, BE, CH, FR, DE, GB, GR, IT, LI, LU, NL, SE**

1. An endogenous calcium channel modulating substance characterized in that said substance is obtained from mammalian tissue selected from brain, pituitary gland, stomach or blood by extracting said tissue with a solution of about 1 normal HCl, 1% trifluoroacetic acid, 5% formic acid and 1% NaCl; on gel filtration with a Waters Protein-Pak® 125 column, said substance has the characteristics of peptides having a molecular weight of 18,000 to 25,000 daltons; elutes from a $C_{18}$ reverse phase column at approximately 31-33% acetonitrile using gradient elution in 0.1% trifluoroacetic acid; has activity which elutes from Bio Gel HT® hydroxylapatite between 0.1 M and 0.2 M sodium phosphate (pH 7); has a molecular weight on sodium dodecylsulfate gels of 18,000 to 20,000; inhibits the binding of tritiated PN 200-110 to cardiac and brain membranes; the activity is stable to boiling; is sensitive to a protease from Streptomyces griseus (from Boehringer--Mannheim Biochemicals, Indianapolis, Indiana USA, Catalog No. 165-921); inhibits the slowly inactivating calcium channel in rat GH3 anterior pituitary cells in a time and voltage-dependent manner when added to the inside of the cell; and, the inhibitory activity of said substance on dihydropyridine receptor binding is reversed by 10 mM $Ca^{2+}$ or by dilution in the presence of 200 mM KCl.

2. The endogenous substance of Claim 1 wherein said mammalian tissue is brain.

3. An endogenous calcium channel modulating substance characterized in that said substance is obtained from mammalian tissue selected from brain, pituitary gland, stomach or blood by extracting said tissue with a solution of about 1 normal HCl, 1% trifluoroacetic acid, 5% formic acid and 1% NaCl; on gel filtration with a Waters Protein-Pak® 125 column, said substance has the characteristics of peptides having a molecular weight of 18,000 to 25,000 daltons; elutes from a $C_{18}$ reverse phase column at approximately 31-33% acetonitrile using gradient elution in 0.1% trifluoroacetic acid; has activity which elutes from Bio Gel HT® hydroxylapatite between 0.1 M and 0.2 M sodium phosphate (pH 7); has a molecular weight on sodium dodecylsulfate gels of 18,000 to 20,000; inhibits the binding of tritiated PN 200-110 to cardiac and brain membranes; the activity is stable to boiling; is sensitive to a protease from Streptomyces griseus (from Boehringer -Mannheim Biochemicals, Indianapolis, Indiana USA, Catalog No. 165-921); inhibits the slowly inactivating calcium channel in rat GH3 anterior pituitary cells in a time and voltage-dependent manner when added to the inside of the cell; the inhibitory activity of said substance on dihydropyridine binding is reversed by 10 mM $Ca^{2+}$, said endogenous calcium channel modulating substance isolated by a method comprising the steps of:

a) homogenizing said mammalian tissue in an acidic solution to form a homogenate;
b) centrifuging the resultant mixture of step (a);
c) extracting the supernatant from step (b) with petroleum ether;
d) subjecting the aqueous phase from step (c) to ultrafiltration so as to retain a fraction of less than 30,000 daltons;
e) subjecting said fraction to chromatography; and
f) isolating said endogenous calcium channel modulating substance.

4. The endogenous substance of Claim 3 wherein said mammalian tissue is brain.

5. A method for isolating an endogenous calcium channel modulating substance from mammalian tissue selected from brain, pituitary gland, stomach or blood comprising the steps of:

a) homogenizing said mammalian tissue to form a homogenate;
b) centrifuging the resultant mixture of step (a);
c) extracting the supernatant from step (b) with petroleum ether;
d) subjecting the aqueous phase from step (c) to ultrafiltration so as to retain a fraction of less than 30,000 daltons;
e) subjecting said fraction to chromatography; and
f) isolating from said column said endogenous calcium channel modulating substance.

6. The method of Claim 5 wherein said mammalian tissue is brain.

7. An endogenous calcium channel modulating substance characterized in that said substance is obtained from mammalian tissue by extracting said tissue with a solution of 1 normal HCl, 1% trifluoroacetic acid, 5% formic acid and 1% NaCl; elutes from a $C_{18}$ reverse phase column at approximately 31-33% acetonitrile using gradient elution in 0.1% trifluoroacetic acid; has a molecular weight of less than 600 daltons; inhibits the binding of tritiated PN 200-110 to cardiac and brain membranes; the activity is stable to boiling; the inhibitory activity of said substance on dihydropyridine receptor binding is not reversed by 10 mM $Ca^{2+}$ or 4 $\mu$M EDTA; and said substance cross-reacts with antibodies to nitrendipine.

8. The endogenous substance of Claim 7 wherein said mammalian tissue is brain.

9. An endogenous calcium channel modulating substance characterized in that said substance is obtained from mammalian tissue by extracting said tissue with a solution of 1 normal HCl, 1% trifluoroacetic acid, 5% formic acid and 1% NaCl; elutes from a $C_{18}$ reverse phase column at approximately 31-33% acetonitrile using gradient elution in 0.1% trifluoroacetic acid; has a molecular weight of less than 600 daltons; inhibits the binding of tritiated PN 200-110 to cardiac and brain membranes; the activity is stable to boiling; the inhibitory activity of said substance on dihydropyridine binding is not reversed by 10 mM $Ca^{2+}$ or 4 $\mu$M EDTA; and said substance cross-reacts with antibodies to nitrendipine, said endogenous calcium channel modulating substance isolated by a method comprising the steps of:

a) homogenizing said mammalian tissue in an acidic solution to form a homogenate;
b) centrifuging the resultant mixture of step (a);
c) extracting the supernatant from step (b) with petroleum ether;
d) subjecting the aqueous phase from step (c) to ultrafiltration so as to retain a fraction of less than 30,000 daltons;
e) subjecting said fraction to chromatography; and
f) isolating said endogenous calcium channel modulating substance.

10. The endogenous substance of Claim 9 wherein said mammalian tissue is brain.

11. An endogenous calcium channel modulating substance characterized in that said substance is obtained from mammalian stomach by extracting said tissue with a solution of about 9% HCl, 1% trifluoroacetic acid, 5% formic acid and 1% NaCl; on gel filtration with a Bio-Rad Bio-Sil TSK® 250 column in 40% acetonitrile, said substance has the characteristics of a peptide having a molecular weight of 7,000 to 10,000 daltons; elutes from a $C_{18}$ analytical reverse phase $\mu$Bondapak Waters® column at approximately 47-50% acetonitrile using gradient elution in 0.1% trifluoroacetic acid; inhibits the binding of tritiated PN 200-110 to cardiac membranes; reversibily inhibits the slowly-inactivating calcium channel in rat $GH_3$ anterior pituitary cells in a time- and voltage-dependent manner when added to the inside of the cell; and, the inhibitory activity of said substance on calcium channels is reversed by hyperpolarization of the membrane.

12. The endogenous substance of Claim 11 wherein said mammalian stomach is lamb abomasum.

13. An endogenous calcium channel modulating substance characterized in that said substance is obtained from lamb abomasum by extracting said tissue with a solution of about 9% HCl, 1% trifluoroacetic acid, 5% formic acid and 1% NaCl; on gel filtration with a Waters Protein-Pak® 250 column in 40% acetonitrile, said substance has the characteristics of peptides having a molecular weight of 7,000 to 10,000 daltons; elutes from a $C_{18}$ reverse phase column at approximately 47-50% acetonitrile using gradient elution in 0.1% trifluoroacetic acid; inhibits the binding of tritiated PN 200-110 to cardiac membranes; reversibly inhibits the slowly inactivating calcium channel in rat GH3 anterior pituitary cells in a time and voltage-dependent manner when added to the inside of the cell; the inhibitory activity of said substance on calcium channels is reversed by hyperpolarizing the cell membrane, said endogenous calcium channel modulating substance isolated by a method comprising the steps of:

a) homogenizing said mammalian tissue in an acidic solution to form a homogenate;
b) centrifuging the resultant mixture of step (a);
c) extracting the supernatant from step (b) with petroleum ether;
d) subjecting the aqueous phase from step (c) to ultrafiltration so as to retain a fraction of less than 30,000 daltons;
e) subjecting said fraction to chromatography; and
f) isolating said-endogenous calcium channel modulating substance.

14. A method for isolating an endogenous calcium channel modulating substance from lamb abomasum comprising the steps of:

a) homogenizing said mammalian tissue in an acidic solution to form a homogenate;
b) centrifuging the resultant mixture of step (a);
c) extracting the supernatant from step (b) with petroleum ether;
d) subjecting the aqueous phase from step (c) to ultrafiltration so as to retain a fraction of less than 30,000 daltons;
e) subjecting said fraction to chromatography; and
f) isolating said endogenous calcium channel modulating substance.

**Claims for the following Contracting State : ES**

EP 0 261 522 B1

1. A method for isolating an endogenous calcium channel modulating substance from mammalian tissue selected from brain, pituitary gland, stomach or blood comprising the steps of:

   a) homogenizing said mammalian tissue to form a homogenate;
   b) centrifuging the resultant mixture of step (a);
   c) extracting the supernatant from step (b) with petroleum ether;
   d) subjecting the aqueous phase from step (c) to ultrafiltration so as to retain a fraction of less than 30,000 daltons;
   e) subjecting said fraction of chromatography; and
   f) isolating from said column said endogenous calcium channel modulating substance.

2. The method of Claim 1 wherein said mammalian tissue is brain.

3. A method for isolating an endogenous calcium channel medulating substance from lamb abomasum comprising the steps of:

   a) homogenizing said mammalian tissue in an acidic solution to form a homogenate;
   b) centrifuging the resultant mixture of step (a);
   c) extracting the supernatant from step (b) with petroleum ether;
   d) subjecting the aqueous phase from step (c) to ultrafiltration so as to retain a fraction of less than 30,000 daltons;
   e) subjecting said fraction to chromatography; and
   f) isolating said endogenous calcium channel modulating substance.

**Revendications**
**Revendications pour les Etats contractants suivants : AT, BE, CH, DE, FR, GB, GR, IT, LI, LU, NL, SE**

1. Substance endogène à effet de modulation sur les canaux de migration du calcium, caractérisée en ce qu'elle est obtenue à partir d'un tissu de mammifère choisi entre les tissus du cerveau, de l'hypophyse, de l'estomac et le sang, en soumettant ledit tissu à une extraction avec une solution de HCl approximativement 1 Normal, 1 % d'acide trifluoracétique, 5 % d'acide formigue et 1 % de NaCl ; ladite substance, par filtration sur gel au moyen d'une colonne Waters Protein-Pak® 125, présentant les caractéristiques de peptides ayant un poids moléculaire de 18 000 à 25 000 daltons ; présentant une élution d'une colonne à inversion de phase $C_{18}$ à approximativement 31 à 33 % d'acétonitrile au moyen d'une élution en gradient dans 0,1 % d'acide trifluoracétique ; possédant une activité qui présente une élution de l'hydroxy-apatite Bio Gel HT® à une concentration en phosphate de sodium de 0,1 M à 0,2 M (pH 7) ; possédant un poids moléculaire, sur des gels au dodécylsulfate de sodium, de 18 000 à 20 000 ; inhibant la liaison du PN 200-110 tritié aux membranes des tissus du coeur et du cerveau ; présentant une activité stable à l'ébullition ; étant sensible à une protéase de Streptomyces griseus (de Boehringer-Mannheim Biochemicals, Indianapolis, Indiana USA, catalogue n° 165-921) ; inhibant l'inactivation lente des canaux de migration du calcium dans des cellules d'antéhypophyse de rat GH3 d'une manière dépendant du temps et de la tension lors de l'introduction à l'intérieur de la cellule ; et présentant une activité inhibitrice de la liaison aux récepteurs de la dihydropyridine qui est inversée par le $Ca^{2+}$ 10 mM ou par dilution en présence de KCl 200 mM.

2. Substance endogène suivant la revendication 1, dans laquelle le tissu de mammifère est le tissu du cerveau.

3. Substance endogène à effet de modulation sur les canaux de migration du calcium, caractérisée en ce qu'elle est obtenue à partir d'un tissu de mammifère choisi entre les tissus du cerveau, de l'hypophyse, de l'estomac et le sang, en soumettant ledit tissu à une extraction avec une solution de HCl approximativement 1 Normal, 1 % d'acide trifluoracétique, 5 % d'acide formique et 1 % de NaCl ; ladite substance, par filtration sur gel au moyen d'une colonne Waters Protein-Pak® 125, présentant les caractéristiques de peptides ayant un poids moléculaire de 18 000 à 25 000 daltons ; présentant une élution d'une colonne à inversion de phase $c_{18}$ à approximativement 31 à 33 % d'acétonitrile au moyen d'une élution en gradient dans 0,1 % d'acide trifluoracétique ; possédant une activité qui présente une élution de l'hydroxy-apatite Bio Gel HT® à une concentration en phosphate de sodium de 0,1 M à 0,2 M (pH 7) ; possédant un poids moléculaire, sur des gels au dodécylsulfate de sodium, de

13

18 000 à 20 000 ; inhibant la liaison du PN 200-110 tritié aux membranes des tissus du coeur et du cerveau ; présentant une activité stable à l'ébullition ; étant sensible à une protéase de Streptomyces griseus (de Boehringer-Mannheim Biochemicals, Indianapolis, Indiana USA, catalogue n° 165-921) ; inhibant l'inactivation lente des canaux de migration du calcium dans des cellules d'antéhypophyse de rat GH3 d'une manière dépendant du temps et de la tension lors de l'introduction à l'intérieur de la cellule ; et présentant une activité inhibitrice de la liaison aux récepteurs de la dihydropyridine qui est inversée par le $Ca^{2+}$ 10 mM, ladite substance endogène à effet de modulation des canaux de migration du calcium étant isolée par un procédé comprenant les étapes consistant :

a) à homogénéiser ledit tissu de mammifère dans une solution acide pour la formation d'un homogénat ;

b) à centrifuger le mélange résultant de l'étape (a) ;

c) à soumettre le surnageant de l'étape (b) à une extraction avec de l'éther de pétrole ;

d) à soumettre la phase aqueuse de l'étape (c) à une ultrafiltration de manière à retenir une fraction de moins de 30 000 daltons ;

e) à soumettre ladite fraction à une chromatographie ; et

f) à isoler ladite substance endogène à effet de modulation sur les canaux de migration du calcium.

4. Substance endogène suivant la revendication 3, dans laquelle le tissu de mammifère est le tissu du cerveau.

5. Procédé pour isoler une substance endogène, à effet de modulation sur les canaux de migration du calcium, d'un tissu de mammifère choisi entre les tissus du cerveau, de l'hypophyse, de l'estomac et le sang, comprenant les étapes consistant :

a) à homogénéiser ledit tissu de mammifère pour la formation d'un homogénat ;

b) à centrifuger le mélange résultant de l'étape (a) ;

c) à soumettre le surnageant de l'étape (b) à une extraction avec de l'éther de pétrole ;

d) à soumettre la phase aqueuse de l'étape (c) à une ultrafiltration de manière à retenir une fraction de moins de 30 000 daltons ;

e) à soumettre ladite fraction à une chromatographie ; et

f) à isoler de ladite colonne ladite substance endogène à effet de modulation sur les canaux de migration du calcium.

6. Procédé suivant la revendication 5, dans laquelle le tissu de mammifère est le tissu du cerveau.

7. Substance endogène à effet de modulation sur les canaux de migration du calcium, caractérisée en ce qu'elle est obtenue à partir d'un tissu de mammifère en soumettant ledit tissu à une extraction avec une solution de HCl 1 Normal, 1 % d'acide trifluoracétique, 5 % d'acide formique et 1 % de NaCl ; présente une élution d'une colonne à inversion de phase $C_{18}$ à approximativement 31 à 33 % d'acétonitrile au moyen d'une élution en gradient dans 0,1 % d'acide trifluoracétique ; possède un poids moléculaire inférieur à 600 daltons ; inhibe la liaison du PN 200-110 tritié aux membranes des tissus du coeur et du cerveau ; possède une activité stable à l'ébullition ; présente une activité inhibitrice de la liaison aux récepteurs de la dihydropyridine qui n'est pas inversée par le $Ca^{2+}$ 10 mM ou l'EDTA 4 $\mu$m ; ladite substance présentant une réaction croisée avec des anticorps contre la nitrendipine.

8. Substance endogène suivant la revendication 7, dans laquelle le tissu de mammifère est le tissu du cerveau.

9. Substance endogène, à effet de modulation sur les canaux de migration du calcium, caractérisée en ce qu'elle est obtenue à partir d'un tissu de mammifère en soumettant à une extraction ledit tissu avec une solution de HCl 1 Normal, 1 % d'acide trifluoracétique, 5 % d'acide formique et 1 % de NaCl ; présente une élution d'une colonne à inversion de phase $C_{18}$ à approximativement 31 à 33 % d'acétonitrile au moyen d'une élution en gradient dans 0,1 % d'acide trifluoracétique ; possède un poids moléculaire inférieur à 600 daltons ; inhibe la liaison du PN 200-110 tritié aux membranes des tissus du coeur et du cerveau ; possède une activité stable à l'ébullition ; présente une activité inhibitrice de la liaison de la dihydropyridine qui n'est pas inversée par le $Ca^{2+}$ 10 mM ou le EDTA 4 $\mu$M ; et présente une réaction croisée avec des anticorps contre la nitrendipine ; ladite substance endogène, à effet de modulation sur les canaux de migration du calcium, étant isolée par un procédé comprenant les étapes consistant :

a) à homogénéiser ledit tissu de mammifère dans une solution acide pour la formation d'un homogénat ;

b) à centrifuger le mélange résultant de l'étape (a) ;

c) à soumettre le surnageant de l'étape (b) à une extraction avec de l'éther de pétrole ;

d) à soumettre la phase aqueuse de l'étape (c) à une ultrafiltration de manière à retenir une fraction de moins de 30 000 daltons ;

e) à soumettre ladite fraction à une chromatographie ; et

f) à isoler ladite substance endogène à effet de modulation sur les canaux de migration du calcium.

10. Substance endogène suivant la revendication 9, dans laquelle le tissu de mammifère est le tissu du cerveau.

11. Substance endogène, à effet de modulation sur les canaux de migration du calcium, caractérisée en ce qu'elle est obtenue à partir d'un estomac de mammifère, en soumettant à une extraction ledit tissu avec une solution d'approximativement 9 % de HCl, 1 % d'acide trifluoracétique, 5 % d'acide formique et 1 % de NaCl ; ladite substance, par filtration sur gel au moyen d'une colonne BioRad Bio-Sil TSK® 250 dans 40 % d'acétonitrile, présentant les caractéristiques d'un peptide ayant un poids moléculaire de 7000 à 10 000 daltons ; présentant une élution d'une colonne analytique à inversion de phase $C_{18}$ μBondapak Waters® à approximativement 47 à 50 % d'acétonitrile au moyen d'une élution en gradient dans 0,1 % d'acide trifluoracétique ; inhibant la liaison du PN 200-110 tritié aux membranes cardiaques ; inhibant de manière réversible les canaux de migration du calcium, à inactivation lente, présents dans les cellules d'antéhypophyse de rat $GH_3$ d'une manière dépendant du temps et de la tension lors de son introduction dans la cellule ; et présentant une activité inhibitrice sur les canaux de migration du calcium qui est inversée par hyperpolarisation de la membrane.

12. Substance endogène suivant la revendication 11, dans laquelle l'estomac de mammifère est la caillette d'agneau.

13. Substance endogène, à effet de modulation sur les canaux de migration du calcium, caractérisée en ce qu'elle est obtenue à partir de caillette d'agneau en soumettant à une extraction ledit tissu avec une solution à environ 9 % de HCl, 1 % d'acide trifluoracétique, 5 % d'acide formique et 1 % de NaCl ; ladite substance, par filtration sur gel au moyen d'une colonne Waters Protein-Pak® 250 dans 40 % d'acétonitrile, présentant les caractéristiques de peptides ayant un poids moléculaire de 7000 à 10 000 daltons ; présentant une élution d'une colonne à inversion de phase $C_{18}$ à approximativement 47 à 50 % d'acétonitrile au moyen d'une élution en gradient dans 0,1 % d'acide trifluoracétique ; présentant une inhibition de la liaison du PN 200-110 tritié aux membranes du tissu cardiaque ; inhibant de manière réversible les canaux de migration du calcium, à inactivation lente, présents dans les cellules d'antéhypophyse de rat GH3 d'une manière dépendant du temps et de la tension lors de son introduction à l'intérieur de la cellule ; l'activité inhibitrice de ladite substance sur les canaux de migration du calcium étant inversée par hyperpolarisation de la membrane cellulaire, ladite substance endogène à effet de modulation sur les canaux de migration du calcium étant isolée par un procédé comprenant les étapes consistant :

a) à homogénéiser ledit tissu de mammifère dans une solution acide pour la formation d'un homogénat ;

b) à centrifuger le mélange résultant de l'étape (a) ;

c) à soumettre le surnageant de l'étape (b) à une extraction avec de l'éther de pétrole ;

d) à soumettre la phase aqueuse de l'étape (c) à une ultrafiltration de manière à retenir une fraction de moins de 30 000 daltons ;

e) à soumettre ladite fraction à une chromatographie ; et

f) à isoler ladite substance endogène à effet de modulation sur les canaux de migration du calcium.

14. Procédé pour isoler de la caillette d'agneau une substance endogène, à effet de modulation sur les canaux de migration du calcium, comprenant les étapes consistant :

a) à homogénéiser ledit tissu de mammifère dans une solution acide pour la formation d'un homogénat ;

b) à centrifuger le mélange résultant de l'étape (a) ;

c) à soumettre le surnageant de l'étape (b) à une extraction avec de l'éther de pétrole ;

d) à soumettre la phase aqueuse de l'étape (c) à une ultrafiltration de manière à retenir une fraction de moins de 30 000 daltons ;

e) à soumettre ladite fraction à une chromatographie ; et

f) à isoler ladite substance endogène à effet de modulation sur les canaux de migration du calcium.

**Revendications pour l'Etat Contractant suivant: ES**

1. Procédé pour isoler une substance endogène, à effet de modulation sur les canaux de migration du calcium, d'un tissu de mammifère choisi entre les tissus du cerveau, de l'hypophyse, de l'estomac et le sang, comprenant les étapes consistant :

a) à homogénéiser ledit tissu de mammifère pour la formation d'un homogénat ;

b) à centrifuger le mélange résultant de l'étape (a) ;

c) à soumettre le surnageant de l'étape (b) à une extraction avec de l'éther de pétrole ;

d) à soumettre la phase agueuse de l'étape (c) à une ultrafiltration de manière à retenir une fraction de moins de 30 000 daltons ;

e) à soumettre ladite fraction à une chromatographie ; et

f) à isoler de ladite colonne ladite substance endogène à effet de modulation sur les canaux de migration du calcium.

2. Procédé suivant la revendication 1, dans lequel le tissu de mammifère est le tissu du cerveau.

3. Procédé pour isoler de la caillette d'agneau une substance endogène, à effet de modulation sur les canaux de migration du calcium, comprenant les étapes consistant :

a) à homogénéiser ledit tissu de mammifère dans une solution acide pour la formation d'un homogénat ;

b) à centrifuger le mélange résultant de l'étape (a) ;

c) à soumettre le surnageant de l'étape (b) à une extraction avec de l'éther de pétrole ;

d) à soumettre la phase aqueuse de l'étape (c) à une ultrafiltration de manière à retenir une fraction de moins de 30 000 daltons ;

e) à soumettre ladite fraction à une chromatographie ; et

f) à isoler ladite substance endogène à effet de modulation sur les canaux de migration du calcium.

**Patentansprüche**
**Patentansprüche für folgende Vertragsstaaten : AT, BE, CH, DE, FR, GB, GR, IT, LI, LU, NL, SE**

1. Endogener, den Kalziumkanal modulierender Stoff, dadurch gekennzeichnet, daß der genannte Stoff aus Säugetiergewebe erhalten wird, ausgewählt aus Gehirn, Hypophyse, Magen oder Blut, indem man das genannte Gewebe mit einer Lösung von ca. 1 normaler HCl, 1 % Trifluoressigsäure, 5 % Ameisensäure und 1 % NaCl extrahiert, wobei auf Gelfiltration mit einer Waters Protein-Pak ®125-Säule der genannte Stoff die Eigenschaften von Peptiden mit einem Molekulargewicht von 18.000 bis 25.000 Daltons aufweist, aus einer $C_{18}$-Umkehrphasensäule bei annähernd 31-33 % Acetonitril unter Verwendung einer Gradientelution in 0,1 %iger Trifluoressigsäure eluiert, eine Aktivität besitzt, die aus Bio Gel HT ®-Hydroxylapatit mit zwischen 0,1 M und 0,2 M liegendem Natriumphosphat (pH 7) eluiert wird, ein Molekulargewicht an Natriumdodecylsulfatgelen von 18.000 bis 20.000 aufweist, die Bindung von tritiiertem PH 200-110 an Herzmuskel- und Gehirnmembranen inhibiert, die Aktivität gegenüber Erhitzen zum Sieden stabil, gegenüber einer Protease aus Streptomyces griseus (von Boehringer-Mannheim Biochemicals, Indianapolis, Indiana USA, Katalog-Nr. 165-921) empfindlich ist, den sich langsam inaktivierenden Kalziumkanal in Ratten-GH3-Vorderhirnanhangzellen in zeit- und voltabhängiger Weise inhibiert, wenn er dem Innern der Zelle zugefügt wird, und die Inhibitoraktivität des genannten Stoffs auf die Dihydropyridinrezeptorbindung durch 10 mM $Ca^{2+}$ oder durch Verdünnen in der Gegenwart von 200 mM KCl umgekehrt wird.

2. Endogener Stoff gemäß Anspruch 1, worin das genannte Säugetiergewebe Hirngewebe ist.

3. Endogener, den Kalziumkanal modulierender Stoff, dadurch gekennzeichnet, daß der genannte Stoff aus Säugetiergewebe erhalten wird, ausgewählt aus Gehirn, Hypophyse, Magen oder Blut, indem man das genannte Gewebe mit einer Lösung von ca. 1 normaler HCl, 1 % Trifluoressigsäure, 5 % Ameisensäure und 1 % NaCl extrahiert, wobei auf Gelfiltration mit einer Waters Protein-Pak ®125-

16

Säule der genannte Stoff die Eigenschaften von Peptiden mit einem Molekulargewicht von 18.000 bis 25.000 Daltons aufweist, aus einer $C_{18}$-Umkehrphasensäule bei annähernd 31-33 % Acetonitril unter Verwendung einer Gradientelution in 0,1 %iger Trifluoressigsäure eluiert, eine Aktivität besitzt, die aus Bio Gel HT ®-Hydroxylapatit mit zwischen 0,1 M und 0,2 M liegendem Natriumphosphat (pH 7) eluiert wird, ein Molekulargewicht an Natriumdodecylsulfatgelen von 18.000 bis 20.000 aufweist, die Bindung von tritiiertem PN 200-110 an Herzmuskel- und Gehirnmembranen inhibiert, die Aktivität gegenüber Erhitzen zum Sieden stabil, gegenüber einer Protease aus Streptomyces griseus (von Boehringer-Mannheim Biochemicals, Indianapolis, Indiana USA, Katalog-Nr. 165-921) empfindlich ist, den sich langsam inaktivierenden Kalziumkanal in Ratten-GH3-Vorderhirnanhangzellen in zeit- und voltabhängiger Weise inhibiert, wenn er dem Innern der Zelle zugefügt wird, und die Inhibitoraktivität des genannten Stoffes auf die Dihydropyridinbindung durch 10 mM $Ca^{2+}$ umgekehrt wird, wobei der genannte endogene, den Kalziumkanal modulierende Stoff durch ein Verfahren isoliert wird, wobei man:

a) das genannte Säugetiergewebe in einer sauren Lösung homogenisiert, um ein Homogenat zu bilden,
b) die entstandene Mischung aus Stufe (a) zentrifugiert,
c) den Überstand aus Stufe (b) mit Petrolether extrahiert,
d) die wäßrige Phase aus Stufe (c) einer Ultrafiltration unterzieht, um eine Fraktion von weniger als 30.000 Daltons zu erhalten,
e) die genannte Fraktion chromatographiert und
f) den genannten endogenen, den Kalziumkanal modulierenden Stoff isoliert.

4. Endogener Stoff gemäß Anspruch 3, worin das genannte Säugetiergewebe Hirngewebe ist.

5. Verfahren zur Isolierung eines endogenen, den Kalziumkanal modulierenden Stoffes aus Säugetiergewebe, ausgewählt aus Hirn, Hypophyse, Magen oder Blut, wobei man:

a) das genannte Säugetiergewebe homogenisiert, um ein Homogenat zu bilden,
b) die entstandene Mischung aus Stufe (a) zentrifugiert,
c) den Überstand aus Stufe (b) mit Petrolether extrahiert,
d) die wäßrige Phase aus Stufe (c) einer Ultrafiltration unterzieht, um eine Fraktion von weniger als 30.000 Daltons zu erhalten,
e) die genannte Fraktion chromatographiert und
f) den genannten endogenen, den Kalziumkanal modulierenden Stoff aus der Säule isoliert.

6. Verfahren gemäß Anspruch 5, worin das genannte Säugetiergewebe Hirngewebe ist.

7. Endogener, den Kalziumkanal modulierender Stoff, dadurch gekennzeichnet, daß der genannte Stoff aus Säugetiergewebe erhalten wird, indem man das genannte Gewebe mit einer Lösung von 1 normaler HCl, 1 % Trifluoressigsäure, 5 % Ameisensäure und 1 % NaCl extrahiert, wobei er aus einer $C_{18}$-Umkehrphasensäule bei annähernd 31-33 % Acetonitril unter Verwendung einer Gradientelution in 0,1 %iger Trifluoressigsäure eluiert wird, ein Molekulargewicht von weniger als 600 Daltons aufweist, die Bindung von tritiiertem PN 200-110 an Herzmuskel- und Hirnmembranen inhibiert, die Aktivität stabil gegen Erhitzen zum Sieden ist, die Inhibitoraktivität des genannten Stoffes auf die Dihydropyridinrezeptorbindung durch 10 mM $Ca^{2+}$ oder 4 μM EDTA nicht umgekehrt wird und der genannte Stoff eine Kreuzreaktion mit Antikörpern auf Nitrendipin eingeht.

8. Endogener Stoff gemäß Anspruch 7, worin das genannte Säugetiergewebe Hirngewebe ist.

9. Endogener, den Kalziumkanal modulierender Stoff, dadurch gekennzeichnet, daß der genannte Stoff aus Säugetiergewebe erhalten wird, indem man das genannte Gewebe mit einer Lösung von 1 normaler HCl, 1 % Trifluoressigsäure, 5 % Ameisensäure und 1 % NaCl extrahiert, wobei er aus einer $C_{18}$-Umkehrphasensäule bei annähernd 31-33 % Acetonitril unter Verwendung einer Gradientelution in 0,1 %iger Trifluoressigsäure eluiert wird, ein Molekulargewicht von weniger als 600 Daltons aufweist, die Bindung von tritiiertem PN 200-110 an Herzmuskel- und Hirnmembranen inhibiert, die Aktivität stabil gegen Erhitzen zum Sieden ist, die Inhibitoraktivität des genannten Stoffes auf die Dihydropyridinbindung durch 10 mM $Ca^{2+}$ oder 4 μM EDTA nicht umgekehrt wird und der genannte Stoff eine Kreuzreaktion mit Antikörpern auf Nitrendipin eingeht, wobei der genannte endogene, den Kalziumkanal

EP 0 261 522 B1

modulierende Stoff durch ein Verfahren isoliert wird, wobei man:

a) das genannte Säugetiergewebe in einer sauren Lösung homogenisiert, um ein Homogenat zu bilden,

b) die entstandene Mischung aus Stufe (a) zentrifugiert,

c) den Überstand aus Stufe (b) mit Petrolether extrahiert,

d) die wäßrige Phase aus Stufe (c) einer Ultrafiltration unterzieht, um eine Fraktion von weniger als 30.000 Daltons zu erhalten,

e) die genannte Fraktion chromatographiert und

f) den genannten endogenen, den Kalziumkanal modulierenden Stoff isoliert.

10. Endogener Stoff gemäß Anspruch 9, worin das genannte Säugetiergewebe Hirngewebe ist.

11. Endogener, den Kalziumkanal modulierender Stoff, dadurch gekennzeichnet, daß der genannte Stoff aus Säugetiermagen erhalten wird, indem man das genannte Gewebe mit einer Lösung von ca. 9 % HCl, 1 % Trifluoressigsäure, 5 % Ameisensäure und 1 % NaCl extrahiert, wobei der genannte Stoff auf Gelfiltration mit einer Bio-Rad-Bio-Sil TSK ®250-Säule in 40 % Acetonitril die Eigenschaften eines Peptids mit einem Molekulargewicht von 7.000 bis 10.000 Daltons aufweist, aus einer analytischen $C_{18}$-Umkehrphasen-μBondapak Waters ®-Säule bei annähernd 47-50 % Acetonitril unter Einsatz einer Gradientelution in 0,1 %iger Trifluoressigsäure eluiert wird, die Bindung von tritiiertem PN 200-110 an Herzmuskelmembranen inhibiert, den sich langsam inaktivierenden Kalziumkanal in Ratten-$GH_3$-Vorderhirnanhangzellen in zeit- und voltabhängiger Weise reversibel inhibiert, wenn er dem Innern der Zelle zugefügt wird, und die Inhibitoraktivität des genannten Stoffes an Kalziumkanälen durch Hyperpolarisation der Membran umgekehrt wird.

12. Endogener Stoff gemäß Anspruch 11, worin der genannte Säugetiermagen Lammabomasum ist.

13. Endogener, den Kalziumkanal modulierender Stoff, dadurch gekennzeichnet, daß der genannte Stoff aus Lammabomasum erhalten wird, indem man das genannte Gewebe mit einer Lösung von ca. 9 % HCl, 1 % Trifluoressigsäure, 5 % Ameisensäure und 1 % NaCl extrahiert, wobei der genannte Stoff auf Gelfiltration mit einer Bio-Rad-Bio-Sil TSK ®250-Säule in 40 % Acetonitril die Eigenschaften eines Peptids mit einem Molekulargewicht von 7.000 bis 10.000 Daltons aufweist, aus einer analytischen $C_{18}$-Umkehrphasen-μBondapak Waters ®-Säule bei annähernd 47-50 % Acetonitril unter Einsatz einer Gradientelution in 0,1 %iger Trifluoressigsäure eluiert wird, die Bindung von tritiiertem PN 200-110 an Herzmuskelmembranen inhibiert, den sich langsam inaktivierenden Kalziumkanal in Ratten-$GH_3$-Vorderhirnanhangzellen in zeit- und voltabhängiger Weise reversibel inhibiert, wenn er dem Innern der Zelle zugefügt wird, und die Inhibitoraktivität des genannten Stoffes an Kalziumkanälen durch Hyperpolarisation der Zellmembran umgekehrt wird, wobei der genannte endogene, den Kalziumkanal modulierende Stoff durch ein Verfahren isoliert wird, wobei man:

a) das genannte Säugetiergewebe in einer sauren Lösung homogenisiert, um ein Homogenat zu bilden,

b) die entstandene Mischung aus Stufe (a) zentrifugiert,

c) den Überstand aus Stufe (b) mit Petrolether extrahiert,

d) die wäßrige Phase aus Stufe (c) einer Ultrafiltration unterzieht, um eine Fraktion von weniger als 30.000 Daltons zu erhalten,

e) die genannte Fraktion chromatographiert und

f) den genannten endogenen, den Kalziumkanal modulierenden Stoff isoliert.

14. Verfahren zur Isolierung eines endogenen, den Kalziumkanal modulierenden Stoffes aus Lammabomasum, wobei man:

a) das genannte Säugetiergewebe in einer sauren Lösung homogenisiert, um ein Homogenat zu bilden,

b) die entstandene Mischung aus Stufe (a) zentrifugiert,

c) den Überstand aus Stufe (b) mit Petrolether extrahiert,

d) die wäßrige Phase aus Stufe (c) einer Ultrafiltration unterzieht, um eine Fraktion von weniger als 30.000 Daltons zu erhalten,

18

EP 0 261 522 B1

e) die genannte Fraktion chromatographiert und
f) den genannten endogenen, den Kalziumkanal modulierenden Stoff isoliert.

**Patentansprüche für folgenden Vertragsstaat : ES**

1. Verfahren zur Isolierung eines endogenen, den Kalziumkanal modulierenden Stoffes aus Säugetiergewebe, ausgewählt aus Gehirn, Hypophyse, Magen oder Blut, wobei man:

   a) das genannte Säugetiergewebe in einer sauren Lösung homogenisiert, um ein Homogenat zu bilden,
   b) die entstandene Mischung aus Stufe (a) zentrifugiert,
   c) den Überstand aus Stufe (b) mit Petrolether extrahiert,
   d) die wäßrige Phase aus Stufe (c) einer Ultrafiltration unterzieht, um eine Fraktion von weniger als 30.000 Daltons zu erhalten,
   e) die genannte Fraktion chromatographiert und
   f) den genannten endogenen, den Kalziumkanal modulierenden Stoff aus der Säule isoliert.

2. Verfahren gemäß Anspruch 1, worin das genannte Säugetiergewebe Hirngewebe ist.

3. Verfahren zur Isolierung eines endogenen, den Kalziumkanal modulierenden Stoffes aus Lammabomasum, wobei man:

   a) das genannte Säugetiergewebe in einer sauren Lösung homogenisiert, um ein Homogenat zu bilden,
   b) die entstandene Mischung aus Stufe (a) zentrifugiert,
   c) den Überstand aus Stufe (b) mit Petrolether extrahiert,
   d) die wäßrige Phase aus Stufe (c) einer Ultrafiltration unterzieht, um eine Fraktion von weniger als 30.000 Daltons zu erhalten,
   e) die genannte Fraktion chromatographiert und
   f) den genannten endogenen, den Kalziumkanal modulierenden Stoff isoliert.

19

# FIG.1

# FIG.2

# FIG.3

FIG.4